(19) Europäisches Patentamt

European Patent Office

Office européen des brevets

(11) **EP 1 074 218 A1**

(12) **EUROPEAN PATENT APPLICATION**
published in accordance with Art. 158(3) EPC

(43) Date of publication:
07.02.2001 Bulletin 2001/06

(51) Int. Cl.$^7$: **A61B 5/11**, G01N 3/32

(21) Application number: 99917096.2

(86) International application number:
PCT/JP99/02136

(22) Date of filing: 22.04.1999

(87) International publication number:
WO 99/55231 (04.11.1999 Gazette 1999/44)

(84) Designated Contracting States:
AT BE CH CY DE DK ES FI FR GB GR IE IT LI LU
MC NL PT SE

(30) Priority: 24.04.1998 JP 11497798

(71) Applicants:
• ADVANCE CO., LTD.
Tokyo 103 (JP)

• Tamaki, Tamotsu
Minamisaitama-gun, Saitama-ken 345-0835 (JP)

(72) Inventor: TAMAKI, Tamotsu
Minamisaitama-gun Saitama-ken 345-0835 (JP)

(74) Representative:
Goddar, Heinz J., Dr.
FORRESTER & BOEHMERT
Franz-Joseph-Strasse 38
80801 München (DE)

(54) **REPEATED MOMENT LOADING DEVICE**

(57)   A device body for generating a gyroscopic moment is fixed to a human body in its predetermined position to repeatedly apply a moment to the human body. This construction can realize the provision of a repeated moment loading device that can repeatedly apply a moment load to an object through a simple operation.

FIG.2

## Description

FIELD OF THE INVENTION

The invention relates to a repeated moment loading device, which repeatedly applies a moment load to objects, such as human bodies and specimens.

BACKGROUND OF THE INVENTION

In biomechanical studies on the spine, it is common practice to apply a moment load by means of a moment loading device to an upper vertebra of a vertebral column of a specimen dead body to create and measure three-dimensional motion of the upper vertebra; and to determine the relationship between the moment and the three-dimensional motion, thereby elucidating features of the structure of the spine.

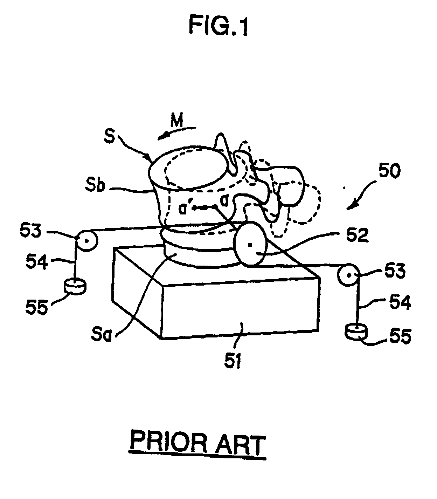

Fig. 1 shows a conventional moment loading device. The conventional moment loading device 50 comprises: a base 51 for fixing a lower vertebra Sa of a specimen S; a disk 51 fixed to a point a of an upper vertebra Sb of the specimen S; a pair of pulleys 53, 53; wires 54 that are extended parallel to each other from the disk 52 and looped on the pulleys 53,53 in respective directions opposite to each other. Weights 55, 55 are mounted as dead loads respectively on both ends of the wires to apply a moment M to the upper vertebra Sb. This permits one point of the upper vertebra Sb to move to another point a' of the upper vertebra Sb. In this case, measurement of three-dimensional motion of the upper vertebra Sb followed by determination of the relationship between the moment M and the three-dimensional motion can elucidate features of the structure of the spine.

However, according to the conventional moment loading device 50, an attempt to apply a moment opposite to the moment shown in Fig.1 to the upper vertebra Sb makes it necessary to alter the position of the pulleys 53, 53 and the direction of the wires 54. This renders the positioning of the pulleys complicate and poses an additional problem that, since the disk 51 is fixedly mounted onto the upper vertebra Sb, the disk cannot be easily detached or attached.

SUMMARY OF THE INVENTION

Accordingly, it is an object of the invention to provide a repeated moment loading device that enables a moment load to be repeatedly applied to an object through a simple operation.

For realizing the above object, according to a feature of the invention, a repeated moment loading device for repeatedly applying a moment load to an object comprises:

a plurality of generation means for generating a plurality of gyroscopic moments:

a supporting member that has a predetermined face and supports the plurality of generation means; and

fixing means for fixing the predetermined face of the supporting member to the object in its predetermined position.

Further, for realizing the above object, according to another feature of the invention, a repeated moment loading device for repeatedly applying a moment load to an object, comprises:

generation means for generating a gyroscopic moment; and

fixing means for fixing the generation means to the object in its predetermined position.

Still further, for realizing the above object, according to still another feature of the invention, a repeated moment loading device for helping a movement of a human body by applying rotating moments, a rotating direction of which periodically changes, to the human body, comprises:

rotating moment generating means for generating the rotating moments at a predetermined rotating face;

mounting means for mounting the rotating moment generating means on a predetermined mounting face such that the predetermined rotating face coincides with one of first to third faces orthogonal to one another; and

fixing means for fixing the rotating moment generating means mounted on the predetermined mounting face to the human body.

It is preferable that the mounting means mounts the rotating moment generating means on the predetermined mounting face by determining a X-Y plane, a Y-Z plane, and a Z-X plane as the first to third faces respectively, in which a front direction of a standing human body is a X axis, a aide direction of the human body is a Y axis and a vertebra of the human body is Z axis of three-dimensional coordinates, respectively.

Further, it is preferable that the fixing means is a fixing belt provided with the predetermined mounting face, and that the fixing belt is fixed around chest, waist, wrist, etc..

Still further, the rotating moment generating means may be mounted on the predetermined mounting face by screws, male-female fitting, etc. to be detached and attached easily. According to this structure, the rotating face of the rotating moment generating means can coincide with one of the X-Y plane, Y-Z plane, and Z-X plane easily.

BRIEF DESCRIPTION OF THE DRAWINGS

Fig. 1 is a perspective view of a conventional moment loading device.

Fig. 2 is an exploded perspective view of a repeated moment loading device according to the first preferred embodiment of the invention.

Fig. 3 is a block diagram showing the body of the repeated moment loading device according to the first preferred embodiment of the invention.

Fig. 4 is a diagram showing the principle of generating first and second gyroscopic moments according to the first preferred embodiment of the invention.

Figs. 5(a), 5(b), and 5(c) are diagrams illustrating mounting of an device body onto a seat plate wherein the first mounting face faces the seat plate, the second mounting face faces the seat plate, and the third mounting face faces the seat plate.

Fig. 6 is a block diagram showing the body of the repeated moment loading device according to the second preferred embodiment of the invention.

DESCRIPTION OF THE PREFERRED EMBODIMENTS

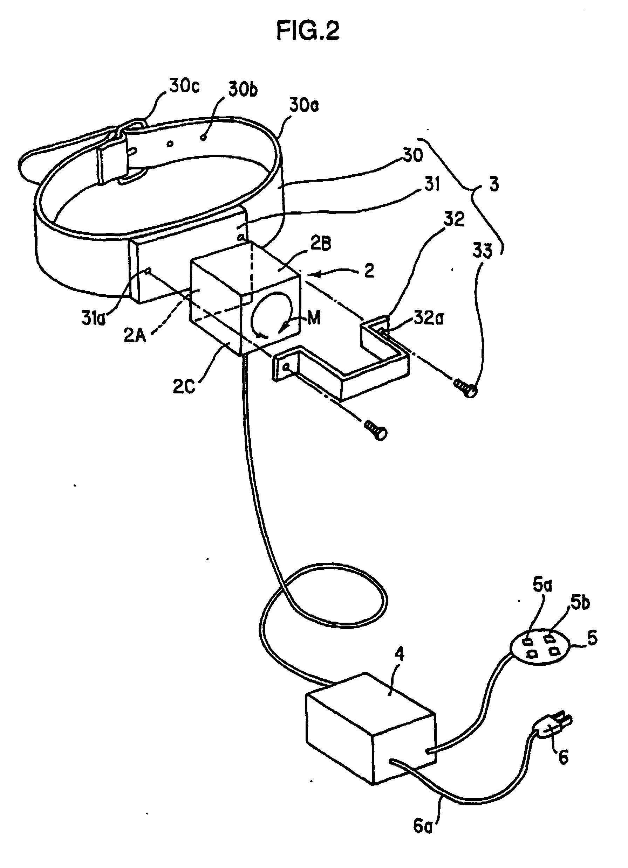

Fig. 2 shows a repeated moment loading device according to the first preferred embodiment of the invention. This repeated moment loading device 1 comprises: an device body 2 which has a first mounting face 2A, a second mounting face 2B, a third mounting face 2C orthogonal to one another and repeatedly generates a moment M around an axis orthogonal to the first mounting face 2A; a fixture 3 for fixing the device body 2 to the human body P as an object in its predetermined position; a controller 4 for controlling the device body 2; an actuator 5 having various switches including a power switch 5a for turning a power supply ON/OFF and a speed selection switch 5b for selecting the speed of the moment M; and a plug 6 for feeding electricity through a power cable 6a to each portion of the device 1.

The fixture 3 comprises a belt 30, a seat plate 31 having a female screw 31a, a hat type mounting tool 32 having a mounting hole 32a, and a bolt 33 threadedly engaged with the female screw 31a of the seat plate 31. The belt 30 has a plurality of fixing holes 30b and comprises a belt body 30a mounted on the seat plate 31 and a metal fitting 30c locked in any one of the fixing holes 30b.

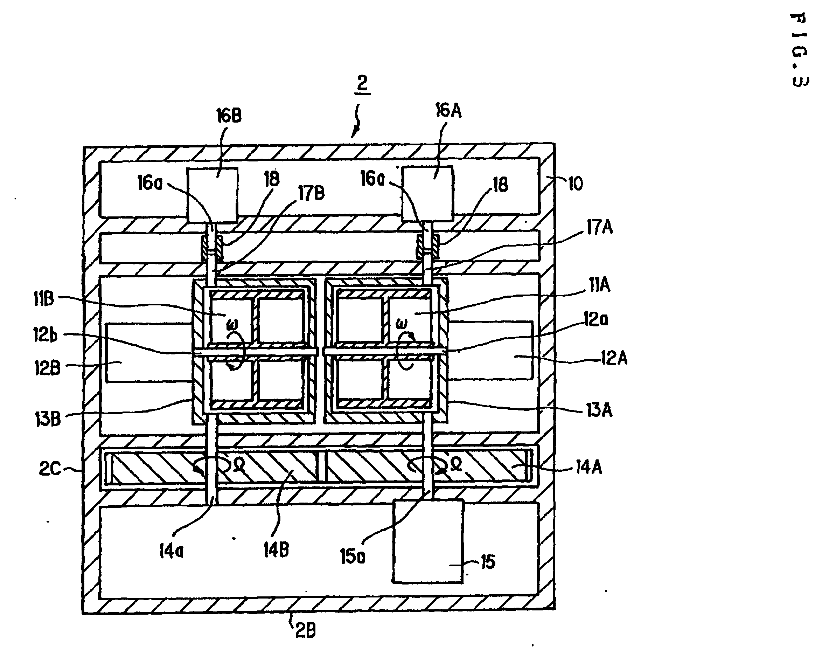

Fig. 3 shows a device body 2. This device body 2 comprises: a substantially box-shaped body case 10; a first rotor 11A fixed to a motor shaft 12a as a first rotor shaft; a second rotor 11B fixed to a motor shaft 12b as a second rotor shaft; a first rotor motor 12A for rotating the first rotor 11A at an angular velocity of ω; a second rotor motor 12B for rotating the second rotor 11B at an angular velocity of ω in a direction opposite to that in the first rotor 11A; a first gimbal 13A which is rotatably supported on the body case 10 by a motor shaft 15a as a first gimbal shaft and a supporting shaft 17A, and supports a first rotor 11A and a motor 12A for the first rotor; a second gimbal 13B which is rotatably supported on the body case 10 by a gear shaft 14a as a second gimbal shaft provided parallel to the first gimbal shaft and a supporting shaft 17B, and supports a second rotor 11B and a motor 12B for the second rotor; a gimbal motor 15 for rotating the first gimbal 13A and the second gimbal 13B through a pair of gears 14A, 14B in mutually opposite directions at an angular velocity of Ω; a first slip ring 16A which has a rotating shaft 16a linked to the supporting shaft 17A through a coupling 18 and feeds electricity to the motor 12A for the first rotor; and a second slip ring 16B which has a rotating shaft 16a linked to the supporting shaft 17B through a coupling 18 and feeds electricity to the motor 12B for the second rotor.

This device body 2 is constructed so that a first gyroscopic moment is generated by the first rotor 11A rotated at an angular velocity of ω around the motor shaft 12a and the first gimbal 13A rotated at an angular velocity of Ω around the motor shaft 15a, orthogonal to the motor shaft 12a, and a supporting shaft 17A, and a second gyroscopic moment is generated by the second rotor 11B rotated at an angular velocity of ω around the motor shaft 12b and the second gimbal 13B rotated at an angular velocity of Ω around gear shaft 14a , orthogonal to the motor shaft 12b, and a supporting shaft 17B so that the second gyroscopic moment is in mirror image relation with the first gyroscopic moment.

The actuator 4 controls the rotating speed of the rotor motors 12A, 12B and the gimbal motor 15 so that the load of the moment M is applied to the human body P in a cycle corresponding to the speed selected by the operator through a speed selection switch 5b.

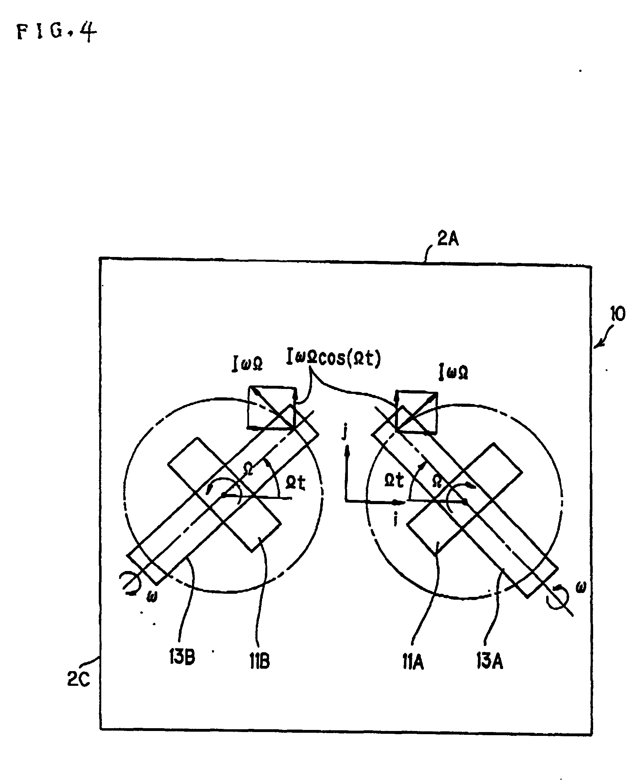

Fig. 4 shows the principle of generating the first and second gyroscopic moments. The rotation of the first and second rotors 11A, 11B and the rotation of the first and second gimbals 13A, 13B result in the generation of the first gyroscopic moment IωΩ and the second gyroscopic moment IωΩ. The total moment M of the first and second gyroscopic moments is expressed by the following equation (1):

$$M = 2I\omega\Omega\cos(\Omega t)j \qquad (1)$$

wherein

I: the moment of the rotors 11A and 11B
ω: the angular velocity of the rotors 11A and 11B
Ω: the angular velocity of the gimbals 13A and 13B
T: the time
J: a unit vector which has a direction shown in the drawing.
I: a unit vector that is orthogonal to j and has a direction shown in the drawing.

As is apparent from the equation (1), since the component force in the direction of the gyroscopic moment $I\omega\Omega$ having both components i and j is offset, the moment M only in the direction of j is obtained as the sum of the two moments. The direction j alternately changes upward and downward with the elapse of time as shown in Fig. 3. Therefore, the direction of the total moment M is alternately changed to the anti-clockwise and clockwise directions as viewed from the first mounting face 2A side. For example, in the equation (1), when I = 17200 (gr*cm$^2$), $\omega$ = 4500 (rpm), and $\Omega$ = 60 (rpm), the moment M = 10.16 cos $(2\pi t)$j(Nm) .

Next, the operation of the device 1 will be explained also in conjunction with Fig. 4.

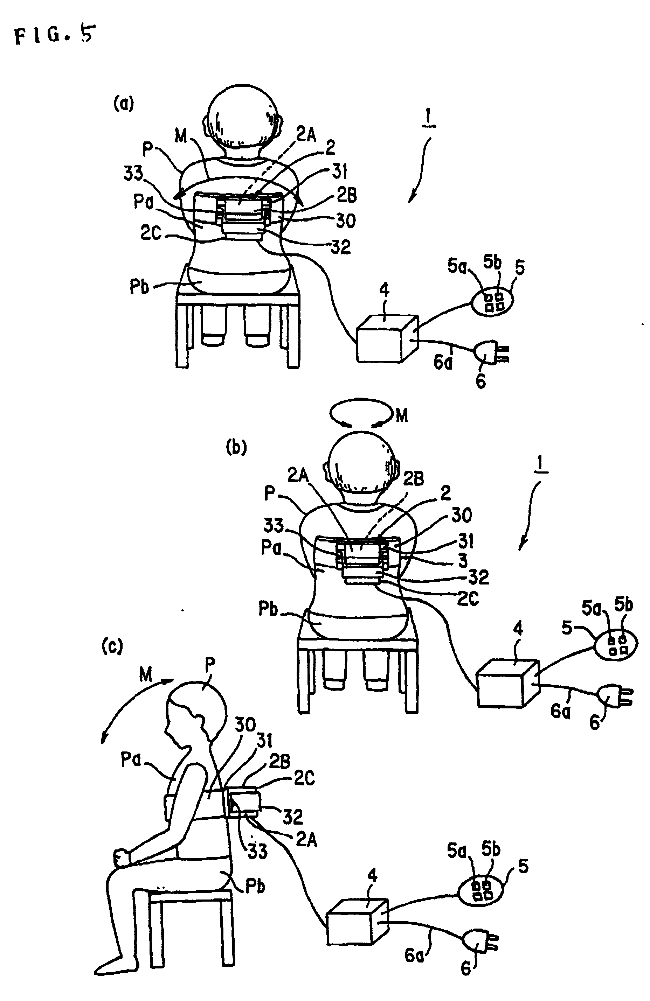

Figs. 5(a), 5(b) and 5(c) show mounting of an device body onto a seat plate 31 wherein the first mounting face 2A faces the seat plate (Fig. 5A), the second mounting face 2B faces the seat plate (Fig. 5B), and the third mounting face 2C faces the seat plate (Fig. 5C).

(1) Lateroflexional motion

For example, when the body P is subjected to lateroflexional motion, as shown in Fig. 4(a), the operator tightens a belt 30 around the human body P in its region of chest Pa. The device body 2 is mounted on the seat plate 31 by means of a mounting tool 32 so that the first mounting face 2A faces the seat plate 31. The plug 6 is inserted in an outlet, and the power switch 5a of the actuator 5 is turned on. This permits electricity to be fed from the plug 6 to each section of the device 1 through the power cable 6a. Subsequently, as soon as the operator selects the speed through the speed selection switch 5b, the controller 4 controls the rotating speed of the rotor motors 12A, 12B and the gimbal motor 15 so that the load of the moment M is applied to the human body P in a cycle corresponding to the speed selected by the speed selection switch 5b. The first and second rotor motors 12A, 12B rotate the first and second rotors 11A, 11B at an angular velocity of $\omega$ in mutually opposite directions by control of the controller 4. The gimbal motor 15 rotates the first and second gimbals 13A, 13B at an angular velocity of $\Omega$ in mutually opposite directions through the gears 14A, 14B by control of the controller 4. The device body 2 generates a moment M around an axis orthogonal to the first mounting face 2A. In Fig. 4 (a), the direction of the moment M is alternately changed to the anti-clockwise and clockwise directions. This moment M is repeatedly applied to the human body P, and the human body P is subjected to lateroflexional motion.

(2) Circumnutation

For example, when the circumnutation of the human body P is contemplated, as shown in Fig. 4(b), the device body 2 is mounted on the seat plate 31 so that the second mounting face 2B faces the seat plate 31. When the rotors 11A, 11B and the gimbals 13A, 13B are rotated in the same manner as described above, a moment M is generated around an axis orthogonal to the first mounting face 2A. This moment M is repeatedly applied to the human body P, causing circumnutation of the human body P.

(3) Flexional extension motion

For example, when the flexional extension motion of the human body P is contemplated, as shown in Fig. 4(c), the device body 2 is mounted on the seat plate 31 so that the third mounting face 2C faces the seat plate 31. When the rotors 11A, 11B and the gimbals 13A, 13B are rotated in the same manner as described above, a moment M is generated around an axis orthogonal to the first mounting face 2A. This moment M is repeatedly applied to the human body P to create the flexion-extension motion of the human body P.

The first preferred embodiment of the invention offers the following effects.

(i) What is needed is only to fix the device body 2, for generating the moment M, to the human body P in its predetermined position. Therefore, the moment M can be repeatedly applied to the human body P by simple operation.

(ii) Mounting of the device body 2 onto vertebral columns, joints (such as knee, elbow, shoulder, thigh, wrist, and ankle region) and other regions of the human body P followed by repeated application of the moment M permits diagnosis, treatment (rehabilitation), and shaping-up of the region.

(iii) Since two gimbals 13A, 13B are rotated by one gimbal motor 15, the construction can be simplified. This can realize a reduction in size (100 x 100 x 100 mm or less) of the device body 2 and a reduction in weight (not more than 1 kg). Therefore, a burden at the time of mounting on the human body P can be reduced, and application of unnecessarily high load to the human body P can be avoided.

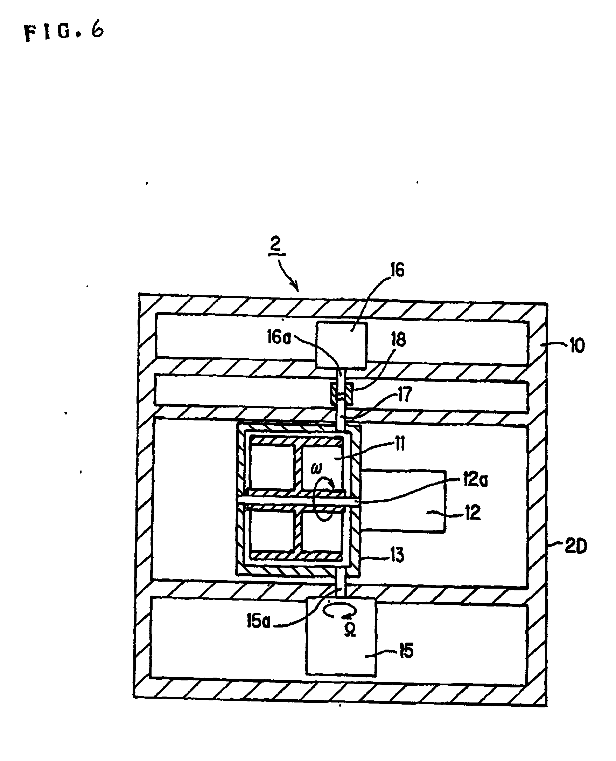

Fig. 6 shows a repeated moment loading device according to the second preferred embodiment of the invention. The repeated moment loading device according to the second preferred embodiment is the same as the repeated moment loading device according to the first preferred embodiment, except that one gyroscopic moment is generated. Specifically, the device body 2 comprises: a substantially box-shaped body case 10; a rotor motor 12 for rotating a rotor 11 fixed to a motor shaft 12a at an angular velocity of $\omega$; a gimbal 13 which is rotatably supported to the body case 10 by a motor shaft 15a and a supporting shaft 17 and supports the rotor 11 and the rotor motor 12; a gimbal motor 15 for rotating the gimbal 13 at an angular velocity of $\Omega$; and a slip ring 16 which has a rotating shaft 16a linked to the supporting shaft 17 through a coupling 18 and feeds electricity to the rotor motor 12.

Next, the operation of the repeated moment loading device having the above construction according to the second preferred embodiment of the invention will be explained. The operator tightens a belt 30 around the human body P in its predetermined position. The device body 2 is mounted on the seat plate 31 by means of a mounting tool 32 so that the motor shaft 15a as the gimbal shaft and the mounting face 2D parallel to the supporting shaft 17 face the seat plate 31. The plug 6 is inserted in an outlet, and the power switch 5a of the actuator 5 is turned on. This permits electricity to be fed from the plug 6 to each section of the device 1 through the power cable 6a. Subsequently, as soon as the operator selects the speed through the speed selection switch 5b, the controller 4 controls the rotating speed of the rotor motor 12 and the gimbal motor 15 so that the load of the moment M is applied to the human body P in a cycle corresponding to the speed selected by the speed selection switch 5b. The rotor motor 12 rotates the rotor 11 at an angular velocity of $\omega$ by control of the controller 4. The gimbal motor 15 rotates the gimbal 13 at an angular velocity of $\Omega$. Rotation of the rotor 11 and the gimbal 13 results in generation of a gyroscopic moment. This causes a moment M to be repeatedly applied to the human body P in its predetermined position.

According to the second preferred embodiment of the invention, what is needed is only to fix the device body 2 for generating the gyroscopic moment to the human body P in its predetermined position. Therefore, the moment M can be repeatedly applied to the human body P by simple operation. Further, as with the first preferred embodiment, according to the second preferred embodiment of the invention, mounting of the device body 2 onto the human body P in its predetermined region followed by repeated application of the moment M permits diagnosis, treatment (rehabilitation), and shaping-up of the region. Furthermore, a reduction in size and a reduction in weight can reduce a burden at the time of mounting on the human body P. This can avoid the application of unnecessarily high load to the human body P.

The invention is not limited to the above preferred embodiments only, and various modifications and variations are possible. For example, the repeated moment loading device may be constructed so that three or more gyroscopic moments are generated.

Further, although gears have been used as the transmission mechanism in the first preferred embodiment, a belt may be used. This can reduce the weight.

Further, in the first preferred embodiment, the mounting face may be one inclined to the axis of the moment M. This can realize combined motion of the human body P, such as lateroflexion/circumnutation, lateroflexion/flexional extension, and circumnutation/flexional extension.

As is apparent from the foregoing description, according to the repeated moment loading device of the invention, mere fixation of gyroscopic moment generation means, for generating a gyroscopic moment, to an object in its predetermined position permits a moment load to be repeatedly applied to the object through a simple operation.

**Claims**

1. A repeated moment loading device for repeatedly applying a moment load to an object comprising:

    a plurality of generation means for generating a plurality of gyroscopic moments;
    a supporting member that has a predetermined face and supports the plurality of generation means; and
    fixing means for fixing the predetermined face of said supporting member to said object in its predetermined position.

2. The repeated moment loading device according to claim 1, wherein said plurality of generation means comprise: first generation means for generating a first gyroscopic moment; and second generation means for generating a second gyroscopic moment, said second generation means being in mirror image relation with said first gyroscopic moment.

3. The repeated moment loading device according to claim 2, wherein said first generation means comprises a first gimbal supported on said supporting member so as to be rotatable around a first gimbal shaft, a first rotor supported on said first gimbal so as to be rotatable around a first rotor shaft orthogonal to said first gimbal shaft, and a motor for said first rotor that is mounted on said first gimbal and rotates said first rotor around said first rotor shaft at a predetermined angular velocity for said rotor,

    said second generation means comprises a second gimbal that is supported on said supporting member so as to be rotatable around a second gimbal shaft provided parallel to said first gimbal shaft, a second rotor supported on said second gimbal so as to be rotatable around a second rotor shaft orthogonal to said second gimbal shaft, and a motor for said second rotor that is mounted on said second gimbal and rotates said second rotor around said second rotor shaft in a direction opposite to that in said first rotor at said predetermined angular velocity for said rotor, and

said first and second generation means comprises a gimbal motor for generating torque for rotating said first and second gimbals, and a transmission mechanism that transmits said torque from said gimbal, motor to said first and second gimbals to rotate said first and second gimbals in mutually opposite directions at said predetermined angular velocity for said gimbal.

4. The repeated moment loading device according to claim 2 wherein said predetermined face is a first face orthogonal to said axis of said total gyroscopic moment of said first gyroscopic moment and said second gyroscopic moment, a second face orthogonal to said first face, or a third face orthogonal to said first and second faces.

5. The repeated moment loading device according to claim 2, wherein said predetermined face in a face inclined to said axis of said total gyroscopic moment of said first gyroscopic moment and said second gyroscopic moment.

6. A repeated moment loading device for repeatedly applying a moment load to an object, comprising:

generation means for generating a gyroscopic moment; and
fixing means for fixing said generation means to said object in its predetermined position.

7. A repeated moment loading device for helping a movement of a human body by applying rotating moments, a rotating direction of which periodically changes, to the human body, comprising:

rotating moment generating means for generating said rotating moments at a predetermined rotating face;
mounting means for mounting said rotating moment generating means on a predetermined mounting face such that said predetermined rotating face coincides with one of first to third faces orthogonal to one another; and
fixing means for fixing said rotating moment generating means mounted on said predetermined mounting face to the human body.

8. The repeated moment loading device according to claim 7, wherein said mounting means mounts said rotating moment generating means on said predetermined mounting face by determining a X-Y plane, a Y-Z plane, and a Z-X plane as said first to third faces respectively, in which a front direction of a standing human body is a X axis, a side direction of the human body is a Y axis and a vertebra of the human body is Z axis of three-dimensional coordinates, respectively.

9. The repeated moment loading device according to claim 8, wherein said fixing means is a fixing belt provided with said predetermined mounting face.

10. The repeated moment loading device according to claim 9, wherein said fixing belt is fixed around chest, waist, wrist, etc..

11. The repeated moment loading device according to claim 7, wherein said rotating moment generating means is mounted on said predetermined mounting face by screws, male-female fitting, etc. to be detached and attached easily.

# FIG.1

**PRIOR ART**

# FIG.2

FIG.3

## FIG.4

# FIG. 5

(a)

(b)

(c)

FIG. 6

# INTERNATIONAL SEARCH REPORT

| International application No. |
|---|
| PCT/JP99/02136 |

**A. CLASSIFICATION OF SUBJECT MATTER**
Int.Cl$^6$ A61B5/11, G01N3/32

According to International Patent Classification (IPC) or to both national classification and IPC

**B. FIELDS SEARCHED**

Minimum documentation searched (classification system followed by classification symbols)
Int.Cl$^6$ A61B5/103-5/11, G01N3/32-3/34, G01M7/02

| Documentation searched other than minimum documentation to the extent that such documents are included in the fields searched | | | |
|---|---|---|---|
| Jitsuyo Shinan Koho | 1922-1996 | Toroku Jitsuyo Shinan Koho | 1994-1999 |
| Kokai Jitsuyo Shinan Koho | 1971-1999 | Jitsuyo Shinan Toroku Koho | 1996-1999 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 10-2827, A (Mitsubishi Heavy Industries,Ltd.), 6 January, 1998 (06. 01. 98), Par. No. [0015] ; Fig. 7 (Family: none) | 1-11 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 8 June, 1999 (08. 06. 99) | 22 June, 1999 (22. 06. 99) |

| Name and mailing address of the ISA/ Japanese Patent Office | Authorized officer |
|---|---|
| Facsimile No. | Telephone No. |

Form PCT/ISA/210 (second sheet) (July 1992)